(19) Europäisches Patentamt / European Patent Office / Office européen des brevets

(11) **EP 4 488 673 B1**

(12) **FASCICULE DE BREVET EUROPEEN**

(45) Date de publication et mention de la délivrance du brevet:
**01.10.2025 Bulletin 2025/40**

(21) Numéro de dépôt: **24186586.4**

(22) Date de dépôt: **04.07.2024**

(51) Classification Internationale des Brevets (IPC):
**G01N 27/90** *(2021.01)*

(52) Classification Coopérative des Brevets (CPC):
(C-Sets disponibles)
**G01N 15/0606; B22F 10/28; B22F 10/34; B22F 10/73; B22F 12/90; B33Y 10/00; B33Y 40/00; B33Y 40/20; G01N 15/0656; G01N 27/90; G01N 33/20;** G01N 2015/0096 (Cont.)

(54) **METHODE ET DISPOSITIF DE CARACTERISATION D'UNE POUDRE METALLIQUE**

VERFAHREN UND VORRICHTUNG ZUR CHARAKTERISIERUNG EINES METALLPULVERS

METHOD AND DEVICE FOR CHARACTERIZING A METAL POWDER

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorité: **05.07.2023 FR 2307173**

(43) Date de publication de la demande:
**08.01.2025 Bulletin 2025/02**

(73) Titulaire: **Commissariat à l'Energie Atomique et aux Energies Alternatives
75015 Paris (FR)**

(72) Inventeurs:
• **SERGEEVA-CHOLLET, Natalia
91191 Gif-sur-Yvette (FR)**
• **SADDOUD, Romain
91191 Gif-sur-Yvette (FR)**
• **PERLIN, Kévyn
91191 Gif-sur-Yvette (FR)**
• **GARANDET, Jean-Paul
91191 Gif-sur-Yvette (FR)**

(74) Mandataire: **Atout PI Laplace
Immeuble Up On
25 Boulevard Romain Rolland
CS 40072
75685 Paris Cedex 14 (FR)**

(56) Documents cités:
FR-A1- 3 087 270        US-A1- 2006 127 267
US-A1- 2016 339 519

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

(52) Classification Coopérative des Brevets (CPC):
(Cont.)

C-Sets
B22F 2999/00, B22F 3/003, B22F 2203/03,
B22F 10/34;

B22F 2999/00, B22F 3/003, B22F 2203/03,
B22F 10/73;
B22F 2999/00, B22F 10/34, B22F 10/73,
B22F 2202/05;
B22F 2999/00, B22F 12/90, B22F 10/34,
B22F 2202/05

## Description

## Domaine de l'invention

La présente invention concerne le domaine de la caractérisation de poudre métallique, et s'intéresse plus particulièrement au contrôle par courants de Foucault de la qualité d'une poudre d'acier austénitique résiduelle après une opération de fabrication additive.

## Etat de la technique

Le terme de Fabrication Additive (FA) désigne selon la norme NF E 67-001, « l'ensemble des procédés permettant de fabriquer couche par couche par ajout de matière un objet physique à partir d'un objet numérique ». Ce terme regroupe des dizaines d'appellations de technologies de fabrication, classées en sept catégories de procédés selon la norme NF ISO 17296-2 Juin 2015.

Les procédés par fusion sur lit de poudres (PBF) pour « Powder Bed Fusion » en anglais, où la poudre est fondue et resolidifiée localement, possèdent le point commun de procéder à une fusion partielle ou totale d'une poudre, provenant généralement de matériaux métalliques, céramiques ou plastiques.

Les procédés PBF diffèrent selon la nature de la source d'énergie employée pour produire la fusion, qui peut être par exemple un laser (le procédé est alors appelé L-PBF pour « Laser-Power Bed Fusion ») ou un faisceau d'électrons (le procédé est alors appelé EB-PBF pour « Electron Beam-Power Bed Fusion »).

En fabrication additive de pièces métalliques, notamment par des procédés PBF, L-PBF, EB-PBF, la quantité de poudre utilisée lors de la fabrication d'une pièce dépasse rarement les 30% du volume total de la poudre employée pour le processus complet de fabrication.

Dans certains cas, pour ne prendre aucun risque sur une prochaine opération, la poudre non fusionnée et récupérée, est directement jetée ou est soumise à un procédé de recyclage en voie longue (fusion + atomisation), afin de n'utiliser que de la poudre neuve et assurer la qualité des futures pièces fabriquées. Ceci conduit à une augmentation du coût de la pièce.

Au vu de la quantité de poudre restante après une opération de fabrication de pièce, il est ainsi intéressant, au moins pour des raisons de coûts, de pouvoir réutiliser la poudre métallique restante dans de futures fabrications.

Cependant, la réutilisation de la poudre ne peut se prévoir que pour une poudre résiduelle dont la qualité est efficacement contrôlée. En effet, pendant le processus de fabrication, une partie de la poudre a été exposée à des températures élevées, et ceci peut avoir changé les propriétés de la poudre.

En particulier, en fabrication L-PBF, certaines particules au voisinage de cordons solidifiés peuvent être entraînées par le flux gazeux et fondre sous l'effet du laser (ce sont des éjectas chauds).

Dans le cas de poudres d'aciers austénitiques (qui est un type spécifique d'alliage d'acier inoxydable contenant de l'austénite), en fonction de leurs cinétiques de refroidissement, ces particules peuvent resolidifier sous forme de ferrite. Et de par leurs propriétés magnétiques, les particules ferritiques posent un problème dans le contrôle de l'étalement de la poudre pour les opérations de fabrication additive.

Dès lors, une réutilisation directe d'une telle poudre usagée pourrait avoir comme effets néfastes de dégrader les propriétés d'une pièce qui serait fabriquée avec.

Selon le standard ASTM F3184 (« Standard Specification for Additive Manufacturing Stainless Steel Alloy (UNS S31603) with Powder Bed Fusion »), la réutilisation de poudre est autorisée pour un nombre non limité de fois, sous condition que la qualité de la pièce finale fabriquée reste constante.

Aussi, il est fondamental de contrôler la qualité d'une poudre résiduelle après une utilisation en fabrication additive, en vue de réutiliser tout ou partie de la poudre restante pour une nouvelle fabrication de pièce.

Un article de T. Delacroix, et al. "Influence of powder recycling on 316L stainless steel feedstocks and printed parts in laser powder bed fusion" (Addit. Manuf. 2021, 50, 102553) étudie la présence de ferrite dans différents types de poudres: poudre neuve ou poudre recyclée jusqu'à 15 fois. La méthode de référence permettant de caractériser finement la présence de ferrite est la diffraction d'électrons rétrodiffusés (EBSD) ou « Electron BackScatter Diffraction » en anglais.

Cette méthode peut fournir des informations très précises sur la composition de la poudre et l'orientation cristalline des particules. Cependant, elle demande une préparation spécifique des échantillons et nécessite un temps important pour les mesures et les analyses. Elle ne permet par ailleurs que de traiter des quantités très limitées de matière, et n'est donc pas adaptée à un environnement industriel.

A une échelle un peu plus importante, la Diffraction aux Rayons X (DRX) permet une quantification des phases observées avec un moindre besoin de préparation des échantillons, mais les quantités de matière investiguées restent limitées, et la technique DRX n'est pas non plus adaptée à un environnement industriel.

US2006/0127267 A1 divulgue un dispositif de mesure sans contact de la conductivité électrique d'une poudre non métallique à l'aide de courants de Foucault. Il comprend une chambre d'échantillonnage diélectrique cylindrique et une bobine d'inductance fonctionnant comme un capteur de courant de Foucault est enroulée sur la surface extérieure de la chambre.

Il existe donc un besoin d'une solution appropriée pour caractériser une poudre métallique résiduelle après une opération de fabrication de pièce en fabrication additive, et notamment pour déterminer la présence de particules de ferrite dans une telle poudre.

La présente invention répond à ce besoin.

**Résumé de l'invention**

Un objet de la présente invention concerne un procédé et un dispositif de caractérisation d'une poudre métallique. Le dispositif selon la présente invention est défini dans la revendication 1.

Le procédé selon la présente invention est défini dans la revendication 8.

Un but de la présente invention est de remédier aux inconvénients précités des approches connues, en proposant un procédé, et un dispositif associé, de détermination de la présence de particules de ferrite dans une poudre métallique résiduelle après une opération en fabrication additive selon un procédé par fusion sur lit de poudres.

Le principe général de l'invention consiste à contrôler par des mesures par courants de Foucault, l'apparition de particules ferritiques dans une poudre d'acier inoxydable récupérée après une opération en fabrication additive selon un procédé par fusion sur lit de poudres.

Le dispositif de l'invention combine l'utilisation d'un récipient d'accueil de la poudre à caractériser, récipient ayant un volume déterminé selon les caractéristiques de l'application de fabrication additive, avec l'utilisation d'un capteur de mesures à courants de Foucault.

Avantageusement, le récipient est conçu pour faire des mesures sur un échantillon de poudre suffisamment représentatif, permettant d'évaluer la qualité de la poudre récupérée.

Avantageusement, le capteur à courants de Foucault selon l'invention, est optimisé pour être sensible aux changements de propriétés électromagnétiques d'une poudre en présence de particules de ferrite.

L'invention trouvera des applications avantageuses dans de nombreux domaines techniques tels que les industries de l'aéronautique, du spatial, de l'automobile ou du nucléaire, pour ne citer que ces exemples.

Pour obtenir les résultats recherchés, il est proposé un dispositif de caractérisation d'une poudre selon la revendication indépendante de dispositif.

Le dispositif comprend :

- un récipient de poudre configuré pour accueillir un échantillon de poudre résiduelle restante après une opération de fabrication additive par un procédé par fusion sur lit de poudres, le récipient étant calibré dans ses dimensions pour contenir ledit échantillon et comprenant un couvercle supérieur composé d'un film de recouvrement et d'un couvercle d'étanchéité ;
- un appareil émetteur/récepteur à courants de Foucault ayant une bobine dont le diamètre est dimensionné en fonction des dimensions du récipient de poudre et configuré pour émettre à la surface de l'échantillon de poudre au travers du film de recouvrement, des signaux électromagnétiques dans une gamme de fréquences de travail prédéfinies ; et

- un système d'acquisition et de pilotage configuré pour :

  - mesurer pour chaque fréquence de travail, des valeurs d'impédance aux bornes d'une bobine de réception de l'appareil émetteur/récepteur à courants de Foucault ; et
  - évaluer la qualité de la poudre restante, en fonction des valeurs d'impédance mesurées pour l'échantillon de poudre.

Selon des modes de réalisation alternatifs ou combinés :

- Le récipient de poudre comprend un corps principal comprenant au moins une chambre d'accueil de poudre, et un bloc d'appui permettant le plaquage de la poudre, la chambre d'accueil de poudre étant dimensionnée pour recevoir un échantillon de poudre représentatif de la poudre restante après fabrication.
- Le récipient de poudre comprend un dispositif d'évacuation d'air configuré pour aspirer l'air contenu dans le récipient de poudre.
- Le récipient est réalisé dans une gamme matériaux non métalliques, dont notamment du Delrin (polyoxyméthylène POM), du plexiglas (polyméthacrylate de méthyle), de l'acrylique (polyacrylonitrile PAN), du Mylar (film polyester PET), du caoutchouc.
- L'appareil émetteur/récepteur à courants de Foucault comprend un capteur composé d'une bobine (qui peut être la même pour les fonctions d'excitation et de réception) sans amplification en mode absolu, le diamètre de la bobine étant dimensionné en fonction des dimensions du récipient de poudre.
- L'appareil émetteur/récepteur à courants de Foucault comprend un capteur composé d'une bobine (qui peut être la même pour les fonctions d'excitation et de réception) dont le nombre de couches et le nombre de spires est prédéfini pour assurer que la fréquence de résonance de la bobine est plus élevée que la gamme de fréquences de travail du capteur.
- Le système d'acquisition et de pilotage comprend un impédancemètre pour mesurer les valeurs d'impédance.

L'invention adresse aussi un procédé de caractérisation d'une poudre résiduelle restant après une opération de fabrication additive par un procédé par fusion sur lit de poudres, selon la revendication indépendante de procédé. Le procédé est mis en œuvre pour un échantillon de poudre résiduelle contenu dans un récipient de poudre calibré dans ses dimensions pour contenir ledit échantillon et comprenant un couvercle supérieur composé d'un film de recouvrement et d'un couvercle d'étanchéité. Le procédé comprend des étapes consistant à :

- activer par un capteur à courants de Foucault CF ayant une bobine dont le diamètre est dimensionné en fonction des dimensions du récipient de poudre, l'émission de signaux électromagnétiques dans une gamme de fréquences de travail prédéfinies, à la surface dudit échantillon de poudre, au travers du film de recouvrement ;
- mesurer, pour chaque fréquence de travail, l'impédance aux bornes de la bobine de réception du capteur CF; et
- évaluer la qualité de la poudre restante, en fonction des valeurs d'impédance mesurées pour l'échantillon de poudre.

Selon des modes de réalisation alternatifs ou combinés :

- L'étape d'activation du capteur CF est faite dans une gamme de fréquences de travail entre 2 MHz à 10 MHz.
- L'étape d'activation du capteur CF est faite dans une gamme de fréquences de travail entre 4 MHz et 5 MHz.
- L'étape d'évaluer la qualité de la poudre restante comprend une étape consistant à comparer les mesures d'impédance à des valeurs d'impédance prédéfinies, et à quantifier la présence de particules de ferrite dans l'échantillon de poudre en fonction d'écarts entre les valeurs mesurées et les valeurs prédéfinies.
- L'étape d'évaluer la qualité de la poudre restante, comprend une étape consistant à définir un ratio acceptable de particules de ferrite présentes dans l'échantillon de poudre pour conserver la poudre restante pour une prochaine fabrication additive ou non.
- La caractérisation d'une poudre consiste à déterminer la présence de particules de ferrite dans un échantillon de poudre austénitique de type 316L.

**Description des figures**

Différents aspects et avantages de l'invention vont apparaitre en appui de la description d'un mode préféré d'implémentation de l'invention mais non limitatif, avec référence aux figures ci-dessous :

La figure 1 est une représentation schématique d'un récipient à poudre pour effectuer les mesures selon les principes de l'invention dans un mode de réalisation.

La figure 2 est une vue éclatée du récipient à poudre de la figure 1.

La figure 3 est une représentation schématique du dispositif de caractérisation d'une poudre selon un mode de réalisation de l'invention.

La figure 4 illustre les étapes générales du procédé de détermination de la présence de particules de ferrite dans une poudre selon l'invention.

La figure 5a et la figure 5b illustrent respectivement des mesures comparées de la réactance et de la résistance d'une poudre neuve et d'une poudre recyclée.

**Description détaillée de l'invention**

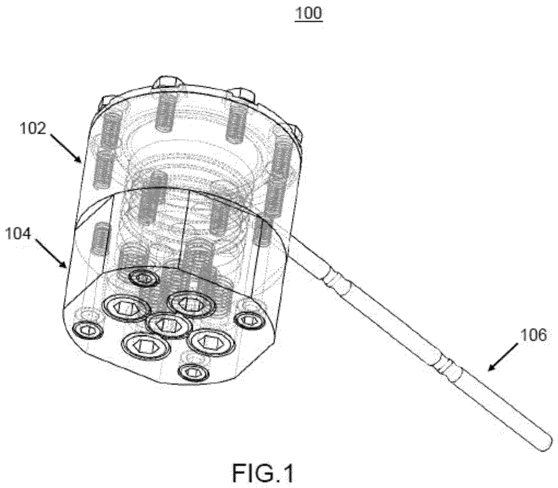

La figure 1 illustre de manière schématique un mode de réalisation d'un récipient selon l'invention, destiné à recevoir un échantillon de poudre, afin d'effectuer des mesures de caractérisation de la poudre selon les principes de l'invention.

Le récipient 100 est réalisé de manière à effectuer des mesures sur une masse de poudre représentative de la poudre existante en fin d'une opération de fabrication additive (on désigne aussi cette poudre comme étant une poudre résiduelle).

Le récipient 100 est principalement composé de deux parties : un corps principal 102 comprenant au moins une chambre d'accueil de poudre, et un bloc d'appui 104 permettant le plaquage de la poudre. La poudre est plaquée sur la partie haute du récipient (recevant un capteur à courants de Foucault) pour éviter d'avoir de l'air entre le capteur et la poudre et ainsi éviter de fausser le résultat de la mesure.

Le récipient 100 est de plus équipé d'un dispositif 106 d'évacuation d'air.

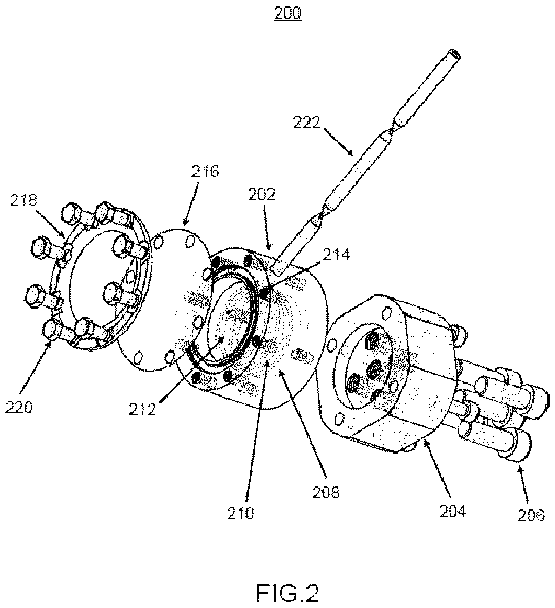

D'autres composants permettant l'assemblage de la structure et le fonctionnement sont décrits en détail en référence à la figure 2.

Avantageusement, un récipient est développé et dimensionné pour une application donnée. En particulier, le diamètre de la chambre d'accueil de la poudre est calculé pour permettre d'accueillir une masse de poudre représentative de la poudre récupérée après une opération de fabrication additive.

Le volume de poudre permettant de faire les mesures selon les principes de l'invention est typiquement de l'ordre de la centaine de grammes. A titre d'exemple non limitatif, pour une masse de poudre comprise entre 50 et 250 grammes, le diamètre de la chambre du récipient peut être ajusté entre 30 et 75 millimètres. L'homme du métier pourra adapter des tailles de récipients à d'autres volumes de poudre tout en conservant les principes de mesures de l'invention.

La figure 2 est une vue éclatée du récipient de la figure 1 dans un mode de réalisation.

Le récipient 200 est composé d'un corps principal 202 qui est réalisé de manière préférentielle dans une matière transparente afin de visualiser la poudre.

Dans une réalisation particulière, le corps principal est réalisé en plexiglas.

Le corps principal 202 vient en appui sur un bloc d'appui 204. Dans un mode de réalisation, le corps principal est vissé sur le bloc d'appui à l'aide d'un jeu de vis 206. Dans une réalisation particulière, quatre vis en inox, de type M10 x 35mm permettent de sceller les deux blocs ensemble.

Le corps principal 202 comprend un piston interne déplaçable 208, avec des joints toriques 210 pour l'étanchéité (deux sur la figure 2), pour pouvoir régler le volume de la poudre 212 à l'intérieur du récipient. Le volume de poudre est réglé en utilisant le bloc d'appui 204 avec les vis 206 en arrière du récipient.

Dans une réalisation particulière, le piston est un piston réalisé en homopolymère d'acétal Delrin (Polyoxyméthylène POM), les joints toriques sont en caoutchouc.

La poudre 212 est bloquée dans la chambre d'accueil par un filtre papier 214 qui sert d'entourage de maintien.

Le récipient 200 comprend de plus une couverture pour enfermer la poudre dans le récipient, via un couvercle supérieur (216, 218) adapté aux tailles des sondes qui seront utilisées pour les mesures.

Le couvercle est composé d'un film de recouvrement 216 et d'un couvercle d'étanchéité 218, le tout scellé au corps principal 202 par un jeu de vis 220.

Dans une réalisation particulière, le film de recouvrement est un film plastique d'une épaisseur comprise entre 50 $\mu$m et 200 $\mu$m.

Dans une réalisation particulière, le couvercle d'étanchéité 218 est réalisé en homopolymère d'acétal Delrin.

Dans une réalisation particulière, le couvercle d'étanchéité 218 est scellé au corps principal par un jeu de huit vis de type M6 x 12mm en polytétrafluoroéthylène (PTFE).

Un dispositif d'évacuation d'air 222 est prévu au niveau de la chambre contenant la poudre, afin d'aspirer l'air et éviter une réaction de la poudre avec l'air. Ceci permet de garantir que les mesures sur la poudre sont faites sans influence de l'air.

Dans un mode de réalisation, le dispositif d'évacuation d'air est un tube accolé au corps principal 202 et dans lequel une seringue (non montrée) est placée afin d'aspirer l'air.

Dans une réalisation particulière, le tube est en acrylique.

L'homme du métier pourra dériver d'autres formes de réalisations particulières à partir des principes généraux décrits. En particulier, des récipients de différentes tailles peuvent être conçus, en tenant compte du principe que les matériaux utilisés ne doivent pas influencer les mesures.

Ainsi, de manière préférentielle, les récipients de poudre et leurs composants peuvent être réalisés dans une gamme matériaux non métalliques : Delrin (polyoxyméthylène POM), plexiglas (polyméthacrylate de méthyle), acrylique (polyacrylonitrile PAN), Mylar (film polyester PET), et caoutchouc.

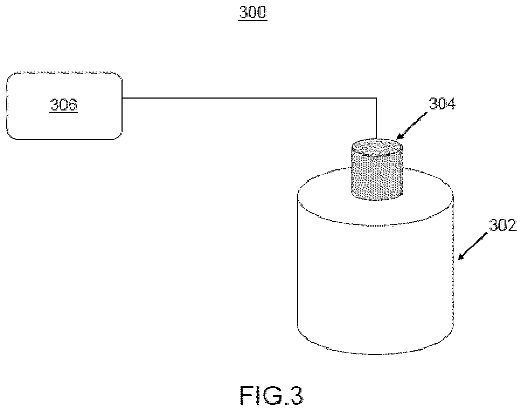

La figure 3 est une représentation schématique du dispositif de caractérisation de poudre de l'invention dans un mode de réalisation.

Généralement, le dispositif de caractérisation de poudre 300 de l'invention est un dispositif simple et compact. Il est composé d'un récipient de poudre 302 calibré pour contenir un échantillon de poudre dont le volume est représentatif d'une poudre récupérée après une opération de fabrication additive.

Le dispositif de caractérisation de poudre comprend aussi un appareil émetteur/récepteur à Courants de Foucault (CF) comprenant un capteur à courant de Foucault 304 optimisé pour l'étude de la poudre.

Le dispositif de caractérisation comprend de plus un système d'acquisition et de pilotage 306 couplé à l'appareil émetteur/récepteur à courants de Foucault, configuré pour déterminer des valeurs d'impédance à partir des signaux reçus lors du contrôle CF, et pour déterminer la présence ou non de particules de ferrite dans l'échantillon de poudre en fonction des valeurs d'impédance mesurées.

Par sa compacité, le dispositif de mesure de l'invention peut être utilisé à proximité d'une machine de fabrication additive ou d'un poste de recyclage de poudre.

Avantageusement par sa simplicité de conception et d'utilisation, le dispositif de mesure de l'invention permet la détection de la présence de ferrite dans un volume de poudres austénitiques résiduelles, sans besoin d'une préparation spécifique de l'échantillon à caractériser.

Un autre avantage du dispositif de l'invention réside dans la rapidité de la caractérisation de la poudre, obtenue par un relevé de mesures par courants de Foucault en surface de la poudre contenue dans le récipient, et par l'analyse immédiate de l'impédance mesurée.

La technique des Courants de Foucault (CF) est basée sur la détection d'un signal électrique émis par un matériau conducteur de l'électricité soumis à un champ électromagnétique variant dans le temps.

Un capteur à courants de Foucault comprend une tête de contrôle qui comporte généralement au moins un circuit à fonction d'émission alimenté en courant alternatif et permettant d'engendrer un champ électromagnétique local, et au moins un récepteur sensible à ce champ électromagnétique.

Le récepteur électromagnétique est souvent constitué d'une bobine réceptrice (éventuellement plusieurs connectées ensemble, par exemple en différentiel) aux bornes de laquelle une force électromotrice de même fréquence que celle du courant alternatif d'alimentation est induite. Le récepteur peut aussi être un capteur de type effet Hall ou encore magnéto-résistif (MR). Cette dernière famille de capteurs regroupe notamment les magnétorésistances anisotropes (AMR), les magnétorésistances géantes (GMR), les magnétorésistances à effet tunnel (TMR), les magnétoimpédances géantes (GMI).

Selon la norme AFNOR NF EN 1330-5, oct. 1998, un transducteur de courants de Foucault est un dispositif physique comportant des éléments d'excitation et des éléments de réception. Dans la suite de la description, le terme capteur CF désigne un tel transducteur de courants de Foucault.

L'agencement et la forme géométrique d'éléments d'émission ou de réception (éléments émetteurs/récepteurs (E/R)) représentent un « motif ». Un motif peut être constitué par des éléments à fonctions émetteur et récepteur séparées, par des éléments regroupant les fonctions E/R, par des éléments ayant une fonction émetteur pour plusieurs récepteurs.

Selon différents modes de réalisation de l'invention, les fonctions d'excitation et de réception peuvent être réalisées de manière différenciée en mode E/R séparé, ou être réalisées en mode E/R confondu, afin de mesurer la réponse en impédance de la bobine.

Lorsque la tête de contrôle d'un capteur à courants de Foucault est disposée au voisinage d'une structure à inspecter ou est déplacée sur la surface d'une telle structure, le circuit émetteur est alimenté en signal sinusoïdal. Un champ électromagnétique de même fréquence est émis dans la structure à inspecter. Il en résulte, aux bornes de la bobine réceptrice, une force électromotrice induite provenant, d'une part, du couplage entre le circuit émetteur et la bobine réceptrice et, d'autre part, du champ magnétique rayonné par les courants induits dans la structure (les courants de Foucault).

Dans les utilisations pour la détection de défauts dans des structures, la circulation des courants induits est modifiée en cas de présence d'une inhomogénéité dans le matériau inspecté. Le récepteur de champ magnétique mesure le champ magnétique résultant de cette modification de trajet des courants induits.

Dans le contexte de l'invention pour la détection de particules de ferrite, la circulation des courants induits est modifiée en fonction de l'impédance du matériau inspecté.

En CF, la sensibilité de la mesure (ou, dit autrement, le rapport signal à bruit) est d'autant meilleure que la distance entre les éléments émetteurs et récepteurs de la tête de contrôle CF et le matériau à inspecter est faible. De plus, lors du déplacement de la tête de contrôle sur le matériau, cette distance (appelée entrefer) doit être la plus constante possible pour éviter que n'apparaisse un biais sur les mesures.

Le capteur CF étant un dispositif assez sensible à l'entrefer, les inventeurs préconisent de travailler en contact avec la surface de la poudre, i.e. la tête du capteur vient en contact de la surface de la poudre au travers du film de recouvrement 216.

Pour concevoir le capteur, i.e. concevoir la bobine, le principe est dans un premier temps d'optimiser le diamètre de la bobine en fonction des dimensions du récipient et de fixer une gamme de fréquences de travail adaptée au matériau à inspecter.

Dans un second temps, il faut optimiser le nombre de couches et le nombre de spires pour assurer que la fréquence de résonance de la bobine soit plus élevée que la fréquence de travail.

Le capteur CF de l'invention est ainsi optimisé pour la détection de la phase ferritique dans la poudre métallique.

En effet, il est nécessaire d'utiliser sur la poudre des fréquences de travail qui soient plus élevées que les fréquences de travail habituellement connues sur les matériaux solides, car la conductivité électrique des poudres est moins élevée que pour les matériaux solides.

De manière spécifique au contexte de l'invention qui est centré sur la détection de ferrite dans les matériaux austénitiques, la gamme de fréquence de travail est comprise dans une plage entre 2 MHz et 10 MHz.

Dans une mise en œuvre particulière, les fréquences de travail sont prises dans une plage entre 3 MHz et 7 MHz.

Dans une autre mise en œuvre, les fréquences de travail sont prises dans une plage entre 4 MHz et 5 MHz.

Les inventeurs ont déterminé que le rapport du diamètre externe de la bobine sur le diamètre du récipient, doit être compris entre 0.2 et 0.35, afin d'effectuer des mesures globales sur la surface du récipient tout en évitant les effets de bord.

Dans un mode de réalisation, la fréquence d'excitation de la bobine est choisie pour être supérieure d'au moins 1 MHz, à la borne supérieure de la gamme de la fréquence de travail, et être préférentiellement supérieure de 2 MHz.

Dans un mode de réalisation particulier, le capteur est composé d'une bobine sans amplification en mode absolu (i.e. émission/ réception confondues). Le diamètre du récipient de poudre est de 45 mm, et le diamètre externe de la bobine est de 12mm. La fréquence de résonance de la bobine est de 7,2 MHz et les mesures d'impédances pour évaluer la présence de ferrite, sont faites dans une gamme de fréquence allant de 4 MHz à 5 MHz.

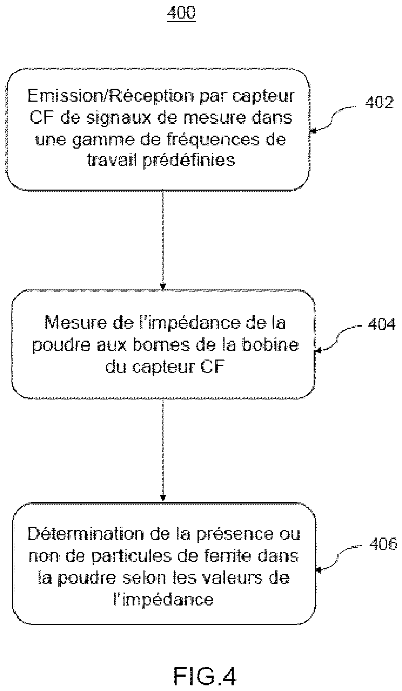

La figure 4 illustre les étapes générales du procédé de caractérisation d'une poudre selon l'invention.

Le procédé est notamment appliqué à la détermination de la présence de particules de ferrite dans une poudre résiduelle, en particulier une poudre austénitique de type 316L.

En effet, une telle poudre, après une opération de fabrication additive selon un procédé par fusion sur lit de poudre (PBF, L-PBF, EB-PBF), peut contenir des particules qui sont resolidifiées sous la forme de ferrite. Ces particules ferritiques peuvent changer la qualité d'étalement de la poudre avant fusion, ce qui peut donner lieu à la formation de défauts en particulier en début de fabrication. Il est donc important de contrôler l'apparition de ferrite dans la poudre résiduelle.

Le principe général du procédé de l'invention 400 consiste à quantifier la présence de particules de ferrite dans un échantillon de poudre représentatif d'une poudre résiduelle restant après une opération de fabrication additive selon un procédé par fusion sur lit de poudre. La détermination de la proportion de particules de ferrite est faite en fonction de la valeur de l'impédance complexe aux bornes de la bobine réceptrice d'un capteur CF qui fonctionne en mode émission/réception (E/R confondu ou séparé), dans une gamme de fréquences de travail optimisées pour la poudre concernée par la mesure.

En effet, en présence de particules de ferrite dans la poudre, les valeurs de la conductivité et de la perméabilité de la poudre sont modifiées, ce qui va engendrer une modification de l'impédance (résistance et réactance), et ce qui permet de révéler la présence de ferrite.

Le procédé de l'invention est opéré sur un dispositif présentant les caractéristiques générales du dispositif décrit en référence à la figure 3, et présentant des caractéristiques spécifiques déterminées selon le contexte de la fabrication additive pour laquelle le contrôle de la poudre est fait.

Ainsi, le dispositif sur lequel est mis en œuvre le procédé de l'invention regroupe un récipient de poudre dont la chambre d'accueil de la poudre a été préalablement dimensionnée et un capteur CF dont les fréquences de travail ont été prédéfinies.

Un échantillon de poudre est posé dans la chambre d'accueil, la poudre est tassée vers le couvercle et l'air pouvant être contenu dans la chambre est aspiré. Le dispositif est préparé.

Dans une première étape 402, le procédé permet d'activer par le capteur CF l'émission de signaux électromagnétiques de mesure dans une gamme de fréquences de travail prédéfinies. La gamme de fréquences prédéfinies est optimisée pour le type de poudre contenue dans le récipient et tient compte de l'exigence de ne pas être trop proche de la fréquence de résonance de la bobine du capteur CF.

La tête du capteur CF est mise au voisinage, au travers du film de recouvrement 216, de la surface de la poudre contenue dans le récipient, pendant toute la durée d'une séquence de mesures (quelques secondes pour relever une centaine de points). L'information recueillie provient d'un volume en sub-surface de l'échantillon de poudre.

Le film de recouvrement a une fonction de sécurité car la matière divisée sous forme de poudre est potentiellement dangereuse en elle-même. De tels films de recouvrement ont des caractéristiques bien connues. Par ailleurs la présence de ce film a également un effet sur la poudre puisqu'il contribue à en rendre la surface plus régulière.

Dans une étape suivante 404, le procédé permet de mesurer l'impédance aux bornes de la bobine réceptrice (force électromagnétique) à l'aide d'un impédancemètre, pour chaque fréquence de la gamme de fréquences prédéfinies, selon des pas de mesure définis.

Puis le procédé dans une étape suivante 406, permet d'évaluer la qualité de la poudre restante, en fonction des valeurs d'impédance mesurées pour l'échantillon de poudre.

En effet, en présence d'une poudre exempte de ferrite, par exemple neuve, avec des valeurs de conductivité $\sigma 1$ et de perméabilité $\mu 1$, l'impédance de la poudre notée $Z_{1poudre,\sigma,\mu_1}$ peut être formulée selon l'équation suivante :

$$Z_{1poudre},\sigma,\mu_1 = R_{poudre},\sigma,\mu_1 + jX_{poudre},\sigma,\mu_1 \,,$$

où $R_{poudre}$ représente la partie réelle et $jX_{poudre}$ la partie imaginaire de l'impédance $Z_{1poudre,\sigma,\mu_1}$.

En cas de présence de particules de ferrite, les valeurs de la conductivité et de la perméabilité de la poudre sont modifiées. Elles sont notées respectivement $\sigma_2$ et $\mu_2$. L'impédance de la poudre avec ferrite est alors formulée selon l'équation suivante :

$$Z_{2poudre},\sigma,\mu_2 = R_{poudre},\sigma_2,\mu_2 + jX_{poudre},\sigma,\mu_2.$$

Selon la quantité de ferrite présente dans l'échantillon mesuré, les valeurs d'impédance $Z_1$ et $Z_2$ pourront être conformes ou différentes.

Dans un mode de réalisation, l'étape 406 d'évaluer la qualité de la poudre restante, comprend une étape consistant à comparer les mesures d'impédance à des valeurs d'impédance prédéfinies, et à quantifier la présence de particules de ferrite dans l'échantillon de poudre, en fonction d'écarts entre les valeurs mesurées et les valeurs prédéfinies.

Dans un mode de réalisation, l'étape 406 d'évaluer la qualité de la poudre restante, comprend une étape consistant à définir un ratio acceptable de particules de ferrite présentes dans l'échantillon de poudre pour conserver la poudre restante pour une prochaine fabrication additive ou non.

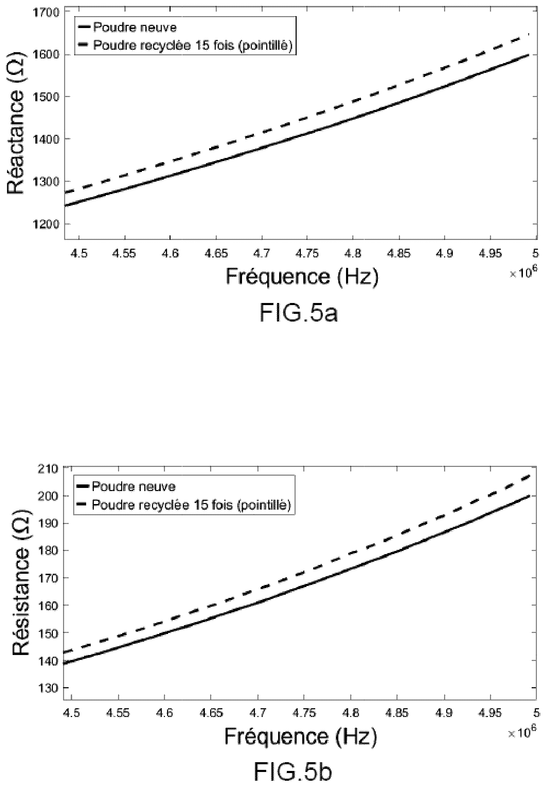

La figure 5a et la figure 5b illustrent respectivement des mesures comparées de la réactance et de la résistance d'une poudre d'acier 316L neuve (en traits pleins) et d'une poudre d'acier 316L recyclée 15 fois (en traits pointillés), mesurées dans une gamme de fréquences de 4,5 MHz à 5 MHz par pas de mesure de 5 kHz. Les courbes en pointillées illustrent que la poudre d'acier recyclée contient des particules de ferrite, les valeurs de réactance et de résistance étant différentes des valeurs respectives pour la poudre neuve.

Ainsi, le procédé de l'invention mis en œuvre sur le dispositif décrit, permet de déterminer, à partir de mesures de valeurs d'impédance pour un échantillon de poudre représentatif d'une poudre résiduelle, si la poudre peut être réutilisée pour une prochaine opération de fabrication additive ou non.

Les valeurs d'impédance mesurées sur l'échantillon sont comparées à des valeurs d'impédance de poudre sans ferrite, par exemple une poudre neuve ou non-recyclée. En cas de non-conformité entre les valeurs, et selon un niveau de pollution aux particules de ferrite qui peut être prédéfini comme étant un seuil acceptable, la poudre dont est extrait l'échantillon, peut être utilisée ou réutilisée pour une prochaine opération de fabrication additive.

**Revendications**

1. Un dispositif (300) de caractérisation d'une poudre résiduelle restant après une opération de fabrication additive par un procédé par fusion sur lit de poudres, le dispositif comprenant :

    - un récipient de poudre (302) pour accueillir un échantillon de ladite poudre résiduelle, le récipient étant calibré dans ses dimensions pour contenir ledit échantillon et comprenant un couvercle supérieur composé d'un film de recouvrement (216) et d'un couvercle d'étanchéité (218) ;
    - un appareil émetteur/récepteur à courants de Foucault (304), ayant une bobine dont le diamètre est dimensionné en fonction des dimensions du récipient de poudre, et configuré pour émettre à la surface de l'échantillon de poudre au travers du film de recouvrement, des signaux électromagnétiques dans une gamme de fréquences de travail prédéfinies ; et
    - un système d'acquisition et de pilotage (306), configuré pour :

        - mesurer pour chaque fréquence de travail, des valeurs d'impédance aux bornes d'une bobine réceptrice de l'appareil émetteur/récepteur à courants de Foucault ; et
        - évaluer la qualité de la poudre restante en fonction des valeurs d'impédance mesurées pour l'échantillon de poudre.

2. Le dispositif selon la revendication 1 dans lequel le récipient de poudre comprend un corps principal (102) comprenant au moins une chambre d'accueil de poudre, et un bloc d'appui (104) permettant le plaquage de la poudre, la chambre d'accueil de poudre étant dimensionnée pour recevoir un échantillon de poudre représentatif de la poudre restante.

3. Le dispositif selon la revendication 1 ou 2 dans lequel le récipient de poudre comprend un dispositif d'évacuation d'air (106, 222) configuré pour aspirer l'air contenu dans le récipient de poudre.

4. Le dispositif selon l'une quelconque des revendications précédentes dans lequel le récipient est réalisé dans une gamme matériaux non métalliques, dont notamment du Delrin (polyoxyméthylène POM), du plexiglas (polyméthacrylate de méthyle), de l'acrylique (polyacrylonitrile PAN), du Mylar (film polyester PET), du caoutchouc.

5. Le dispositif selon l'une quelconque des revendications précédentes dans lequel l'appareil émetteur/récepteur à courants de Foucault comprend un capteur composé d'une bobine sans amplification en mode absolu, le diamètre de la bobine étant dimensionné en fonction des dimensions du récipient de poudre.

6. Le dispositif selon l'une quelconque des revendications précédentes dans lequel l'appareil émetteur/récepteur à courants de Foucault comprend un capteur composé d'une bobine dont le nombre de couches et le nombre de spires est prédéfini pour assurer que la fréquence de résonance de la bobine est plus élevée que la gamme de fréquences de travail du capteur.

7. Le dispositif selon l'une quelconque des revendications précédentes dans lequel le système d'acquisition et de pilotage comprend un impédancemètre pour mesurer les valeurs d'impédance.

8. Procédé (400) de caractérisation d'une poudre résiduelle restant après une opération de fabrication additive par un procédé par fusion sur lit de poudres, le procédé étant mis en œuvre pour un échantillon de ladite poudre résiduelle contenu dans un récipient de poudre calibré dans ses dimensions pour contenir ledit échantillon et comprenant un couvercle supérieur composé d'un film de recouvrement et d'un couvercle d'étanchéité, et comprenant des étapes consistant à :

    - (402) activer par un capteur à courants de Foucault CF ayant une bobine dont le diamètre est dimensionné en fonction des dimensions du récipient de poudre, l'émission de signaux électromagnétiques, dans une gamme de fréquences de travail prédéfinies, à la surface dudit échantillon de poudre au travers du film de recouvrement ;
    - (404) mesurer, pour chaque fréquence de travail, l'impédance aux bornes de la bobine réceptrice du capteur CF; et
    - (406) évaluer la qualité de la poudre restante, en fonction des valeurs d'impédance mesurées pour l'échantillon de poudre.

9. Procédé selon la revendication précédente dans lequel l'étape d'activation du capteur CF est faite dans une gamme de fréquences de travail entre 2 MHz à 10 MHz.

**10.** Procédé selon la revendication 8 dans lequel l'étape d'activation du capteur CF est faite dans une gamme de fréquences de travail entre 4 MHz et 5 MHz.

**11.** Procédé selon l'une quelconque des revendications 8 à 10 dans lequel l'étape 406 d'évaluer la qualité de la poudre restante, comprend une étape consistant à comparer les mesures d'impédance à des valeurs d'impédance prédéfinies, et à quantifier la présence de particules de ferrite dans l'échantillon de poudre, en fonction d'écarts entre les valeurs mesurées et les valeurs prédéfinies.

**12.** Procédé selon l'une quelconque des revendications 8 à 11 dans lequel l'étape 406 d'évaluer la qualité de la poudre restante, comprend une étape consistant à définir un ratio acceptable de particules de ferrite présentes dans l'échantillon de poudre pour conserver la poudre restante pour une prochaine fabrication additive ou non.

**13.** Procédé selon l'une quelconque des revendications 8 à 11 dans lequel la caractérisation d'une poudre consiste à caractériser un échantillon de poudre austénitique de type 316L.

**Patentansprüche**

**1.** Vorrichtung (300) zur Charakterisierung eines Restpulvers, das nach einem additiven Fertigungsvorgang durch ein Pulverbettfusionsverfahren übrig bleibt, wobei die Vorrichtung umfasst:

- einen Pulverbehälter (302) zur Aufnahme einer Probe des Restpulvers, wobei der Behälter in seinen Abmessungen kalibriert ist, um die Probe zu enthalten und einen oberen Deckel umfasst, der aus einer Abdeckfolie (216) und einem Dichtungsdeckel (218) zusammengesetzt ist;
- eine Wirbelstromsende-/-empfängereinrichtung (304) mit einer Spule, von der der Durchmesser in Abhängigkeit der Abmessungen des Pulverbehälters bemessen ist, und die konfiguriert ist, um an der Oberfläche der Pulverprobe durch die Abdeckfolie hindurch elektromagnetische Signale in einem vordefinierten Arbeitsfrequenzbereich zu senden;
- ein Erfassungs- und Steuerungssystem (306), konfiguriert zum:

- Messen, für jede Arbeitsfrequenz, von Impedanzwerten an den Anschlüssen einer Empfängerspule der Wirbelstromsende-/-empfängereinrichtung; und
- Bewerten der Qualität des übrig gebliebenen Pulvers in Abhängigkeit von den für die Pulverprobe gemessenen Impedanzwerten.

**2.** Vorrichtung nach Anspruch 1, wobei der Pulverbehälter einen Hauptkörper (102), der mindestens eine Pulveraufnahmekammer umfasst, und einen Stützblock (104) umfasst, der das Plattieren des Pulvers gestattet, wobei die Pulveraufnahmekammer bemessen ist, um eine Pulverprobe aufzunehmen, die für das übrig gebliebene Pulver repräsentativ ist.

**3.** Vorrichtung nach Anspruch 1 oder 2, wobei der Pulverbehälter eine Entlüftungsvorrichtung (106, 222) umfasst, die zum Absaugen der in dem Pulverbehälter enthaltenen Luft konfiguriert ist.

**4.** Vorrichtung nach einem der vorstehenden Ansprüche, wobei der Behälter aus einem Sortiment nichtmetallischer Materialien angefertigt ist, davon insbesondere Delrin (Polyoxomethylen POM), Plexiglas (Polymethylmethacrylat), Acryl (Polyacrylnitril PAN), Mylar (Polyesterfolie PET), Kautschuk.

**5.** Vorrichtung nach einem der vorstehenden Ansprüche, wobei die Wirbelstromsende-/-empfängereinrichtung einen Sensor umfasst, der aus einer Spule ohne Verstärkung im Absolutmodus zusammengesetzt ist, wobei der Durchmesser der Spule in Abhängigkeit der Abmessungen des Pulverbehälters bemessen ist.

**6.** Vorrichtung nach einem der vorstehenden Ansprüche, wobei die Wirbelstromsende-/-empfängereinrichtung einen Sensor umfasst, der aus einer Spule zusammengesetzt ist, von der die Anzahl an Schichten und die Anzahl an Wicklungen vordefiniert ist, um sicherzustellen, dass die Resonanzfrequenz der Spule höher ist als der Arbeitsfrequenzbereich des Sensors.

**7.** Vorrichtung nach einem der vorstehenden Ansprüche, wobei das Erfassungs- und Steuerungssystem ein Impedanzmessgerät zum Messen der Impedanzwerte umfasst.

**8.** Verfahren (400) zur Charakterisierung eines Restpulvers, das nach einem additiven Fertigungsvorgang durch ein Pulverbettfusionsverfahren übrig bleibt, wobei das Verfahren für eine Probe des Restpulvers durchgeführt wird, das in einem Pulverbehälter enthalten ist, der in seinen Abmessungen kalibriert ist, um die Probe zu enthalten und einen oberen Deckel umfasst, der aus einer Abdeckfolie und einem Dichtungsdeckel zusammengesetzt ist, und die Schritte umfasst bestehend aus:

- (402) Aktivieren, durch einen Wirbelstromsensor (CF-Sensor) mit einer Spule, von der der Durchmesser in Abhängigkeit der Abmessungen des Pulverbehälters bemessen ist, von Remission der elektromagnetischen Signale in einem vordefinierten Arbeitsfrequenzbereich an der Oberfläche der Pulverprobe durch die Abdeckfolie hindurch;
- (404) Messen, für jede Arbeitsfrequenz, der Impedanz an den Anschlüssen der Empfängerspule des CF-Sensors; und
- (406) Bewerten der Qualität des übrig gebliebenen Pulvers in Abhängigkeit von den für die Pulverprobe gemessenen Impedanzwerten.

9. Verfahren nach dem vorstehenden Anspruch, wobei der Aktivierungsschritt des CF-Sensors in einem Arbeitsfrequenzbereich zwischen 2 MHz bis 10 MHz vorgenommen wird.

10. Verfahren nach Anspruch 8, wobei der Aktivierungsschritt des CF-Sensors in einem Arbeitsfrequenzbereich zwischen 4 MHz und 5 MHz vorgenommen wird.

11. Verfahren nach einem der Ansprüche 8 bis 10, wobei der Schritt 406 zum Bewerten der Qualität des übrig gebliebenen Pulvers einen Schritt umfasst, der aus dem Vergleichen der Impedanzmessungen mit vordefinierten Impedanzwerten und dem Quantifizieren der Anwesenheit von Ferritpartikeln in der Pulverprobe in Abhängigkeit von Abweichungen zwischen den gemessenen Werten und den vordefinierten Werten besteht.

12. Verfahren nach einem der Ansprüche 8 bis 11, wobei der Schritt 406 zum Bewerten der Qualität des übrig gebliebenen Pulvers einen Schritt umfasst, der daraus besteht, ein akzeptables Verhältnis von Ferritpartikeln, die in der Pulverprobe vorhanden sind, zu definieren, um das übrig gebliebene Pulver für eine nächste additive oder nicht-additive Fertigung zu bewahren.

13. Verfahren nach einem der Ansprüche 8 bis 11, wobei die Charakterisierung eines Pulvers aus dem Charakterisieren einer austenitischen Pulverprobe vom Typ 316L besteht.

**Claims**

1. A device (300) for characterizing a residual powder remaining after an operation of additive manufacturing by a powder bed fusion process, the device comprising:

   - a powder receptacle (302) for receiving a sample of said residual powder, the receptacle being calibrated in terms of its dimensions to contain said sample and comprising an upper lid composed of a covering film (216) and a sealing lid (218);
   - an eddy current emitting/receiving apparatus (304), having a coil with a diameter dimensioned in accordance with the dimensions of the powder receptacle, and configured to emit to the surface of the powder sample, through the covering film, electromagnetic signals within a range of predefined working frequencies; and
   - an acquisition and control system (306), configured to:

      - measure, for each working frequency, impedance values across the terminals of a receiving coil of the eddy current emitting/receiving apparatus; and
      - evaluate the quality of the remaining powder according to the impedance values measured for the powder sample.

2. The device according to Claim 1, wherein the powder receptacle comprises a main body (102) comprising at least one powder receiving chamber, and a bearing block (104) for pressing the powder, the powder receiving chamber being dimensioned to receive a powder sample representative of the remaining powder.

3. The device according to Claim 1 or 2, wherein the powder receptacle comprises an air evacuation device (106, 222) configured to suck out the air contained in the powder receptacle.

4. The device according to any one of the preceding claims, wherein the receptacle is made from a range of non-metallic materials, including in particular Delrin (polyoxymethylene POM), Plexiglas (polymethyl methacrylate), acrylic (polyacrylonitrile PAN), Mylar (PET polyester film), rubber.

5. The device according to any one of the preceding claims, wherein the eddy current emitting/receiving apparatus comprises a sensor composed of a coil without amplification in absolute mode, the diameter of the coil being dimensioned in accordance with the dimensions of the powder receptacle.

6. The device according to any one of the preceding claims, wherein the eddy current emitting/receiving apparatus comprises a sensor composed of a coil, the number of layers and the number of turns of which is predefined in order to ensure that the resonant frequency of the coil is higher than the range of working frequencies of the sensor.

7. The device according to any one of the preceding claims, wherein the acquisition and control system comprises an impedance meter for measuring the impedance values.

8. A method (400) for characterizing a residual powder remaining after an operation of additive manufacturing by a powder bed fusion process, the method being implemented for a sample of said residual powder contained in a powder receptacle calibrated in terms of its dimensions to contain said sample and comprising an upper lid composed of a covering film and a sealing lid, and comprising the steps of:

> - (402) activating, via an eddy current EC sensor having a coil with a diameter dimensioned in accordance with the dimensions of the powder receptacle, the emission of electromagnetic signals, within a range of predefined working frequencies, to the surface of said powder sample through the covering film;
> - (404) measuring, for each working frequency, the impedance across the terminals of the receiving coil of the EC sensor; and
> - (406) evaluating the quality of the remaining powder according to the impedance values measured for the powder sample.

9. The method according to the preceding claim, wherein the step of activating the EC sensor is carried out within a range of working frequencies between 2 MHz and 10 MHz.

10. The method according to Claim 8, wherein the step of activating the EC sensor is carried out within a range of working frequencies between 4 MHz and 5 MHz.

11. The method according to any one of Claims 8 to 10, wherein step 406 of evaluating the quality of the remaining powder comprises a step of comparing the impedance measurements with predefined impedance values and of quantifying the presence of ferrite particles in the powder sample according to the differences between the measured values and the predefined values.

12. The method according to any one of Claims 8 to 11, wherein step 406 of evaluating the quality of the remaining powder comprises a step of defining an acceptable ratio of ferrite particles present in the powder sample for retaining the remaining powder for a subsequent additive manufacture or not.

13. The method according to any one of Claims 8 to 11, wherein the characterization of a powder consists of characterizing a sample of 316L-type austenitic powder.

100

102

104

106

FIG.1

200

FIG.2

300

306

304

302

FIG.3

<u>400</u>

Emission/Réception par capteur CF de signaux de mesure dans une gamme de fréquences de travail prédéfinies — 402

Mesure de l'impédance de la poudre aux bornes de la bobine du capteur CF — 404

Détermination de la présence ou non de particules de ferrite dans la poudre selon les valeurs de l'impédance — 406

FIG.4

FIG.5a

FIG.5b

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20060127267 A1

**Littérature non-brevet citée dans la description**

- **T. DELACROIX et al.** Influence of powder recycling on 316L stainless steel feedstocks and printed parts in laser powder bed fusion. *Addit. Manuf.*, 2021, vol. 50, 102553